# EUROPEAN PATENT APPLICATION

(11) **EP 4 541 345 A1**
(43) Date of publication of application: **23.04.2025**
(21) Application number: 22754895.5
(22) Date of filing: 17.06.2022
(51) Int. Cl.: A61K 8/35, A61K 8/49, A61Q 19/02

(54) **SYNERGISTIC SKIN DEPIGMENTING COSMETIC COMPOSITION**

(71) Applicant: Mesoestetic Pharma Group, S.L., 08840 Viladecans (Barcelona) (ES)
(72) Inventor: MARTÍNEZ GUTIERREZ, Alfredo, 08840 BARCELONA (ES); BERTRAN JUNQUE, Alexandra, 08840 BARCELONA (ES); PENA RODRÍGUEZ, Eloy, 08840 BARCELONA (ES); LUIS GARCIA, Luis Shotze, 08840 BARCELONA (ES); GONZÁLEZ RODRÍGUEZ, Mari Carmen, 08840 BARCELONA (ES)
(74) Representative: Hernández Hernández, Carlos
(86) International application number: PCT/ES2022/070384
(87) International publication number: WO 2023/105103

(57) **Abstract**

The present invention relates to a cosmetic composition having a skin depigmenting effect which includes, as active ingredient, a combination of apigenin and phloretin, wherein the two components of said combination act synergistically. The invention also relates to the use of the combination of apigenin and phloretin contained in a cosmetic composition for preventing and treating diseases and conditions related to the overproduction or uneven distribution of melanin, wherein said use comprises topically administering an effective quantity of said combination to a patient who needs it, having a synergistic effect.

## Description

### Technical field

The invention relates to a synergistic skin depigmenting cosmetic composition, in addition to the use thereof for depigmenting skin.

More specifically, in a first aspect, the invention provides a cosmetic composition having a skin depigmenting effect which includes, as active ingredient, a combination of apigenin and phloretin, wherein the two components of said combination act synergistically.

In a second aspect, the invention relates to the use of the combination of apigenin and phloretin contained in a cosmetic composition of the invention for the prevention and treatment of diseases and conditions related to the overproduction or uneven distribution of melanin, said use comprising the topical administration of an effective quantity of said combination to a patient who needs it, said combination having a synergistic effect.

### Background art

The colour of the human skin is determined by different factors such as race, sex, phototype, the season of the year and the concentration of melanins in the epidermis, although its complete physiological mechanism has not been fully determined.

The melanocytes present in the basal layer of the epidermis produce melanins within intramelanocyte organelles called melanosomes. Melanins are complex-structure polyphenolic homogeneous biopolymers whose colour varies from brown to black (eumelanin) and from red to yellow (pheomelanin). These melanins (pheomelanins and eumelanins) are transported to nearby keratinocytes to be distributed in the interior thereof and act as a protective shield against ultraviolet radiation.

Melanogenesis is a defensive mechanism of the skin and melanin biosynthesis is stimulated by ultraviolet light. Thus, the physiological function of tanning as a result of the impact of ultraviolet light against the skin is to protect it from these ultraviolet radiations.

Skin pigmentation disorders consisting of melanin overproduction or hypermelanosis and melanin deficiency or hypomelanosis are common. Hypermelanoses appear with greater frequency in certain periods of life and exogenous factors such as excessive exposure to sunlight, inter alia, are usually the cause. These hypermelanoses are characterised by the appearance on the skin, particularly in unprotected areas, of dark and coloured spots which give it a certain degree of visual heterogeneity. It is generally an aesthetic problem. These spots are caused by melanocytic hypertrophy or by a greater accumulation of melanins in the keratinocytes situated on the superficial part of the epidermis.

Similarly, various dysfunctions of melanogenesis due to the effect of exogenous aggressions (for example, hormonal alterations or ageing) trigger the appearance of brown, brownish or blackish spots of melanic origin in the form of melasmas, ephelides, senescence spots, lentigines, etc.

The ability to modify the natural pigmentation of the skin is a widespread desire among the European, Asian and American community, with different nuances such as lightening, whitening, depigmentation, removal of age spots, etc. Therefore, controlling pigmentation through the use of exogenous agents is in great social and commercial demand.

The melanin or melanogenesis formation mechanism has certain complexity and occurs mainly in melanosomes. The biochemical precursor of melanins is the amino acid L-tyrosine, an essential amino acid ubiquitous in the human body.

On the one hand, tyrosinase (EC 1.14 18.1), which exists in the interior of melanosomes, acts as a hydroxylase, transforming L-tyrosine into L-dihydroxyphenylalanin or L-DOPA. Subsequently, tyrosinase enzymatically catalyses the transformation of L-DOPA into dopaquinone, since this enzyme can also act as an oxidase, depending on the substrate. In order to become active, it requires an enzymatic maturation process under the action of certain cellular factors.

Dopaquinone is transformed into dopachrome, which can follow two metabolic pathways: one that leads to the formation of eumelanins, which are black in colour, and another that leads to the formation of pheomelanins, which are yellowish in colour. Both coexist in the skin and their proportion determines its colour or phototype.

Three key enzymes intervene in this melanogenesis process: tyrosinase, limiting enzymatic glycoprotein which is located in the membrane of the melanosomes, Tyrosinase-related protein 1 or TRP 1 and Tyrosinase-related protein 2 or TRP2, glycoproteins that are also located in the melanosome membrane and complement the tyrosinase activity.

Once the melanins are formed in the melanosomes, there must be mechanical transfer of the melanins formed to the epidermal keratinocytes. There is a complex tubular system and cellular factors that contribute to transporting the melanin granules to the melanocyte cell extensions, also called dendrites.

These cytoplasmatic extensions form a network that is in contact with the keratinocytes, forming the so-called melanocyte unit, such that a melanocyte is in contact with up to 36 keratinocytes. The intracellular melanins which are already situated on the melanocyte extensions are transferred to the keratinocytes. The melanin granules are apically arranged in the keratinocytes such as to confer the cell nucleus greater protection against the deleterious effects of UV light.

A powerful stimulus for stimulating melanogenesis is by irradiating the skin with ultraviolet light, which immediately causes an increase in melanocyte cAMP. This increase activates the cAMP/PKA/CREB pathway, which in turn activates the microphthalmia-associated transcription factor or MITF, the main regulator of the genes involved in melanin synthesis (TYR, TRP1, TRP2, etc.), being tyrosinase the main enzyme involved in melanogenesis.

Apigenin, or 5,7-dihydroxy-2-(4-hydroxyphenyl)-4H-1-benzopyran-4-one (4',5,7-trihydroxyflavone), is a well-known natural flavonoid applied in therapeutic compositions due to its beneficial effects as an antioxidant, anti-inflammatory and as a chemical protection agent, in addition to its sedative effect or as a tumoral growth inhibitor. In this respect, see for example patent documents US8377918, CN102441168B or international application WO2005034937.

In the field of cosmetics, apigenin has been the subject of numerous research studies. Thus, for example, in the papers by Yoshihisa Y. and colleagues (The regulation of protein kinase casein kinase II by apigenin is involved in the inhibition of ultraviolet B-induced macrophage migration inhibitory factor-mediated hyperpigmentation. Phytother Res. 2020; 34(6): 1320-1328) studies its anti-melanogenic properties and the relevant molecular action mechanisms. This study shows that, in mice treated with UVB, apigenin inhibits skin hyperpigmentation and the expression of the macrophage migration inhibiting factor (MIF as a key factor related to melanogenesis. In mice keratinocytes, it inhibits the MIF expression and also the related MIF-induced factors (for example, stem cell factor receptor 2 activated by proteinase). In addition to ellagic acid as an inhibitor of casein kinase II (CK2), apigenin suppresses the enzyme activity of CK2 and the CK2 expression induced by UVB and the subsequent phosphorylation of the IKB and MIF expression. These results suggest that apigenin inhibits UVB-induced hyperpigmentation by regulating the CK2-mediated MIF expression in the keratinocytes. The protective role of apigenin against the formation of UVB-induced cyclobutane-pyrimidine dimers (CPD) in human dermal fibroblast cells has also been researched (HDFa) (Britto S.M. and colleagues, Apigenin prevents ultraviolet-B radiation induced cyclobutane pyrimidine dimers formation in human dermal fibroblasts. Mutat Res Genet Toxicol Environ Mutagen. 2017; 821: 28-35), probably due to its sunscreen effect, thereby being considered an effective protector against UV-induced skin damage.

Zhang Y. and colleagues, in "Apigenin induces dermal collagen synthesis via smad2/3 signalling pathway. Eur J Histochem. 2015; 59(2): 2467" describe that apigenin could promote the in vitro and in vivo synthesis of type I and type III dermal fibroblast collagen, which suggests that apigenin could serve as a potential cosmetic and reconstructive agent in anti-ageing skin treatment.

International application WO2020101414 describes a composition for promoting skin regeneration and hair growth containing apigenin, caffeine and ginseng extract, wherein the composition facilitates cell differentiation by means of Wnt/beta-catenin signaling. In this respect, see also Korean patent with publication number 2017-0119757, which describes a cosmetic composition made of apigenin, germanium and amino acids with the same objective.

Furthermore, phloretin (3-(4-hydroxyphenyl)-1-(2,4,6-trihydroxyphenyl)propane-1-one) is a dihydrochalcone, known for its role as a plant metabolite and antineoplastic agent, and for its antioxidant, anti-inflammatory, antimicrobial, antithrombotic and hepatoprotective effects (Aqrokia, V. and colleagues, Pharmacological Aspects and Potential Use of Phloretin: A Systemic Review, Mini Rev Med Chem 2019;19(13):1060-1067). It has been clinically observed that phloretin has antioxidant and skin soothing properties. The antioxidant protection helps to neutralise the free radicals present in the environment. Also, phloretin can be an effective soothing ingredient for problematic skin (Oresajo, C. and colleagues, Protective Effects of a Topical Antioxidant Mixture Containing Vitamin C, Ferulic Acid, and Phloretin against Ultraviolet-induced Photodamage in Human Skin. Journal of Cosmetic Dermatology 7.4, 290-97 (2008); Shin, S. and colleagues, Protective Effects of a New Phloretin Derivative against UVB-Induced Damage in Skin Cell Model and Human Volunteers. IJMS International Journal of Molecular Sciences 15.10, 18919-8940 (2014)). Also, due to the polarity of its chemical structure, allusion is made to the fact that phloretin provides better transdermal administration mechanisms, allowing it to effectively penetrate the skin. Therefore, with its combined antioxidant and soothing properties, phloretin can be an ideal ingredient to implement in products with skin soothing and protective properties (Valenta, C. J. and colleagues, Effect of phloretin on the percutaneous absorption of lignocaine across human skin. Journal of pharmaceutical sciences 90.4, 485-492 (2001)).

The paper by Chen J. and colleagues (Phloretin as both a substrate and inhibitor of tyrosinase: Inhibitory activity and mechanism. Spectrochim Acta A Mol Biomol Spectrosc. 2020; 226: 117642) explains that tyrosinase is the speed-limiting enzyme that controls the production of melanin in the human body, and melanin overproduction can cause a variety of skin disorders. Said paper studies the inhibitory kinetics of phloretin on tyrosinase and its binding mechanism using spectroscopy, molecular coupling, antioxidant assays and chromatography. The results show that phloretin has a great capacity to extinguish the intrinsic fluorescence of tyrosinase, mainly through a static extinguishing procedure, suggesting that a stable phloretin-tyrosinase complex is generated. The results of molecular coupling suggest that the dominant conformation of the phloretin binds to the tyrosinase active site gateway of the tyrosinase. Moreover, antioxidant assays show that phloretin has antioxidant capacity and is capable of reducing, like ascorbic acid, o-dopaquinone to L-DOPA. Curiously, the results of the spectroscopy and chromatography indicate that phloretin is a tyrosinase substrate but also an inhibitor.

CN-113143781 describes a skin care composition having a whitening and moisturising effect composed of active ingredients and adjuvants or acceptable carriers for cosmetic preparations, wherein the active ingredients are composed of phloretin and shikimic acid. CN-113171309 provides a whitening and moisturising cosmetic composition containing phloretin which comprises an active ingredient and an acceptable carrier for a cosmetic preparation, wherein the active ingredient consists of a safe and effective dose of phloretin and soforicoside. The invention finds that the composition of the phloretin and soforicoside has an obvious synergistic skin beautifying effect and, in particular, can synergistically inhibit tyrosinase activity, promote the detachment of melanin-containing keratinocytes, whiten the colour of spots and brighten skin colour. The whitening, moisturising and beautifying composition can be prepared in various preparations in accordance with conventional preparations in the field of cosmetics, such as moisturising essence, face cream or masks and similar.

Patent US9248082B2 discloses a procedure for reducing skin damage induced by radicals which comprises applying to the skin a composition of: (a) phloretin in a quantity of approximately 1% to approximately 2% by weight, based on the total weight of the composition; (b) ferulic acid in a quantity of approximately 0.5% by weight, with respect to the total weight of the composition; (c) ascorbic acid in a quantity of approximately 10% or approximately 15% by weight, based on the total weight of the composition; (d) at least one non-aqueous organic solvent comprising monohydric alcohol selected from the group consisting of ethanol, isopropanol, hexyldecanol, benzylic alcohol, propylic alcohol and isopropylic alcohol, and at least one dihydric alcohol selected from the group consisting of hexylene glicol, diethylene glycol, ethylene glycol, propylene glycol, 1,2-butylene glycol, 1,5-pentanediol, triethylene glycol and dipropylene glycol; (e) water, as a co-solvent; and (f) optionally, vitamin E, in a quantity of approximately 0.02% by weight with respect to the total weight of the composition, wherein said composition does not contain a glycol ether, a monomeric ether and a polymeric ether.

In view of the prior art and the studies carried out on the effect of the different active ingredients on these changes in human epidermal melanocytes and dermal fibroblasts, the Applicant found that, surprisingly, the combination of active ingredients of the invention (apigenin and phloretin) have synergistic depigmenting action, being the action of reducing the melanin levels in human melanocytes of the combination more effective than that of adding the components separately and successively, in addition to inhibiting the key pathways activated in melasma and other skin hyperpigmentations, such as the Wnt and SCF pathways, inter alia (Passeron T. and colleagues Melasma, a photoaging disorder. Pigment Cell Melanoma Res. 2018; 31(4): 461-465; Kim N.H. and colleagues, Reduced MiR-675 in exosome in H19 RNA-related melanogenesis via MITF as a direct target. J Invest Dermatol. 2014; 134(4): 1075-1082).

### Summary of the invention

Therefore, an object of the invention is to provide a cosmetic composition having a skin depigmenting effect that includes, as sole active ingredient, a combination of apigenin and phloretin, wherein the two components of said combination act synergistically, together with the carriers, solvents and/or appropriate excipients for the topical administration thereof. Also object of the invention is the combination of apigenin and phloretin for use in a cosmetic composition described herein.

The components apigenin and phloretin are present in the combination of the invention in an apigenin:phloretin ratio of 1:0.05 to 1:10 (by weight).

Preferably, the combination of apigenin and phloretin is present in the cosmetic composition in a proportion by weight with respect to the total weight of the composition of between 0.02 and 20%.

Another object of the invention is the combination of apigenin and phloretin described herein for use in the manufacture of a cosmetic composition for treating conditions related to the overproduction or uneven distribution of melanin, wherein said use comprises topically administering an effective quantity of said combination to a patient who needs it.

The cosmetic composition of the invention includes, in addition to the previously described combination of apigenin and phloretin, appropriate carriers and excipients for its formulation for the topical administration thereof. Such carriers and excipients can be selected from oily solvents, anhydrous solvents, hydroalcoholic solvents, aqueous solvents, fillers, preservatives, gelling agents, perfumes, surfactants, sunscreens, antioxidants, viscosifying agents, emulsifiers, silicones and any of the ingredients used in the field of cosmetics or dermatology, in the usual concentrations of use, provided that said excipients do not interfere in the depigmenting activity of the described combination. The composition of the invention may be presented in any galenic or cosmetic form commonly used for topical administration, for example in the form of an aqueous, hydroalcoholic, hydroglycolic or oily solution. It can also be included in oil-in-water or water-in-oil emulsions or in a multiple emulsion or be formulated as a serum, a foam or an aerosol or be administered encapsulated in lipid vesicles, for example in transferomes, ethosomes or transethosomes.

### Brief description of drawings

Following is a description of the invention based on examples of embodiment thereof and in reference to the attached figures, wherein:
Figure 1: Shows the results of the synergy testing of the combination of apigenin and phloretin, according to example 1;
Figure 2: Shows gene expression levels of the DKKI (A) and VEGF (B) genes in fibroblasts treated for 24 hours with 10 and 25 µM apigenin, according to example 2;
Figure 3A: Shows gene expression levels of the DCT and PAX3 genes in melanocytes treated with BIO +/- apigenin for 24 hours, according to example 3;
Figure 3B: Shows gene expression levels of the genes DCT, PAX3, PMEL17 and TYRP1 in melanocytes treated with BIO +/- phloretin for 24 hours, according to example 3;
Figure 4A: Shows gene expression levels of the DCT gene in melanocytes treated with CBD +/- apigenin for 24 hours, according to example 4;
Figure 4B Shows gene expression levels of the PAX3 gene in melanocytes treated with CBD +/- apigenin for 24 hours, according to example 4;
Figure 5: Shows miR-675 Levels in melanocytes treated with apigenin or phloretin for 24 hours, according to example 5; and
Figure 6: Shows an *ex vivo* study of the depigmenting effectiveness of the combination of apigenin and phloretin.

### Description of embodiments

The following examples were designed on the basis of conditions favourable to melanogenesis.

### Example 1: Assay of the synergy of the combination of apigenin and phloretin on skin depigmentation in human epidermal melanocytes

3-isobutyl-1-methylxanthine (IBMX) and other cAMP elevators, such as α-melanocyte-stimulating hormone (α-MSH), stimulate melanin production in melanocytes. Intracellular cAMP activates protein kinase A (PKA), which phosphorylates the cAMP-sensitive element binding protein (CRE) (CREB) at residue Ser-133. This phosphorus (p)-CREB up-regulates the microphthalmia-associated transcription factor (MITF) expression, which is crucial for melanocyte differentiation and melanogenesis. In turn, MITF binds to the M-box containing CATGTG motifs in promoter regions of tyrosinase genes or tyrosinase-related proteins (TRPs) for transcriptional activation, leading to increased melanin biosynthesis (Lee, H.D., and colleagues (2011), Manassantin A inhibits cAMP-induced melanin production by down-regulating the gene expressions of MITF and tyrosinase in melanocytes. Experimental Dermatology, 20: 761-763. https://doi.org/10.1111/j.1600-0625.2011.01296.x).

With the aim of assessing whether the combination of the active ingredients apigenin and phloretin have a synergistic effect, human epidermal melanocytes (Cellsystems, # FC-0030) were cultured under different conditions for 48 hours. The conditions of the experiment are as follows:
1) Melanocytes cultured in culture medium (control).
2) Melanocytes cultured in culture medium with 2mM L-tyrosine + 0.5 mM IBMX (3-isobutyl-1-methylxanthine). The stimulus (L-tyrosine + IBMX) was added to these melanocytes every 24 hours until the end of the experiment (stimulated).
3) Melanocytes cultured in culture medium with 2 mM L-tyrosine + 0.5 mM IBMX with 10 µM phloretin + 0.5 µM apigenin every 24 hours successively. In this case, 2 mM L-tyrosine + 0.5 mM IBMX + 10 µM phloretin is added during the first 24 hours, then the culture medium is removed, the cells are washed with PBS and 2mM L-tyrosine + 0.5 mM IBMX + 0.5 µM apigenin is added during a further 24 hours, until completing the 48 hours of the experiment (additive effect).
4) Melanocytes cultured in culture medium with 2 mM L-tyrosine + 0.5 mM IBMX with 10 µM phloretin + 0.5 µM apigenin in combination. In this case, 2 mM L-tyrosine + 0.5 mM IBMX + 10 µM phloretin + 0.5 µM apigenin is added every 24 hours until completing the 48 hours of the experiment (combination effect).

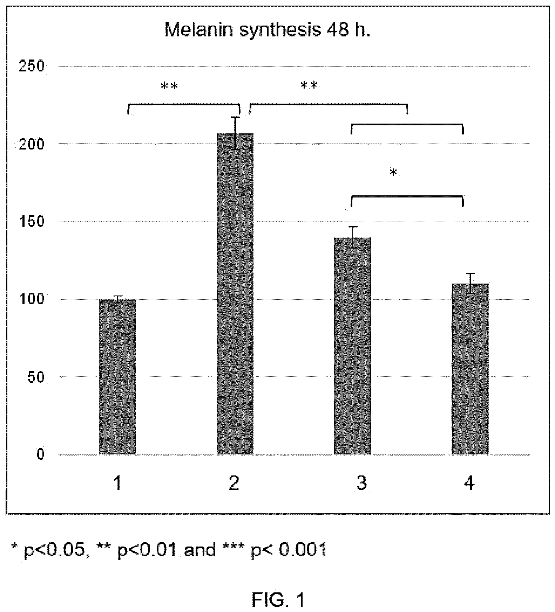

Upon completion of the treatment, the cells were trypsinised and resuspended in 1 M NaOH to extract the melanin from the cells. The absorbance of the resulting solution was measured at 340 nm to quantify the melanin levels of each condition. The results are shown in figure 1.

As can be observed, stimulation with L-tyrosine + IBMX increases the melanin levels to 206% with respect to the non-stimulated control. As regards treatment with the active ingredients, adding each of the active ingredients successively and separately decreases melanin levels to 140%, whereas adding the two active ingredients together in combination decreases melanin levels to 110%. All these changes are statistically significant. Therefore, we can observe that the combination of the active ingredients is more effective than adding each of them successively in decreasing melanin levels in human melanocyte culture.

### Example 2: Assay evaluating the key pigmentation regulation markers in human dermal fibroblasts

The role of dermal fibroblasts in skin pigmentation has been presented both in physiological and pathological skin conditions. In the case of physiological conditions, it was identified that dickkopf 1 (DKK1) plays a role in the hypopigmented phenotype of palmoplantar skin (Yamaguchi et al., 2004); DKK1 is secreted by fibroblasts. As an inhibitor of the canonic Wnt signalling pathway, DKK1 suppresses the function and growth of melanocytes through the regulation of MITF and beta-catenin. The role of fibroblast-derived factors in the regulation of human skin colour has also been identified (Lee AY. Recent progress in melasma pathogenesis. Pigment Cell Melanoma Res. 2015; 28(6): 648-60.)

To assess the levels of key pigmentation regulatory markers in fibroblasts, dermal fibroblasts (Promocell, # C-12302) were cultured for 24 hours with the active ingredient apigenin. After the treatment, the RNA was extracted (Norgen, #17200) and real-time qPCR was performed (Takara, #RR420A) on the DKK1 and VEGF genes.

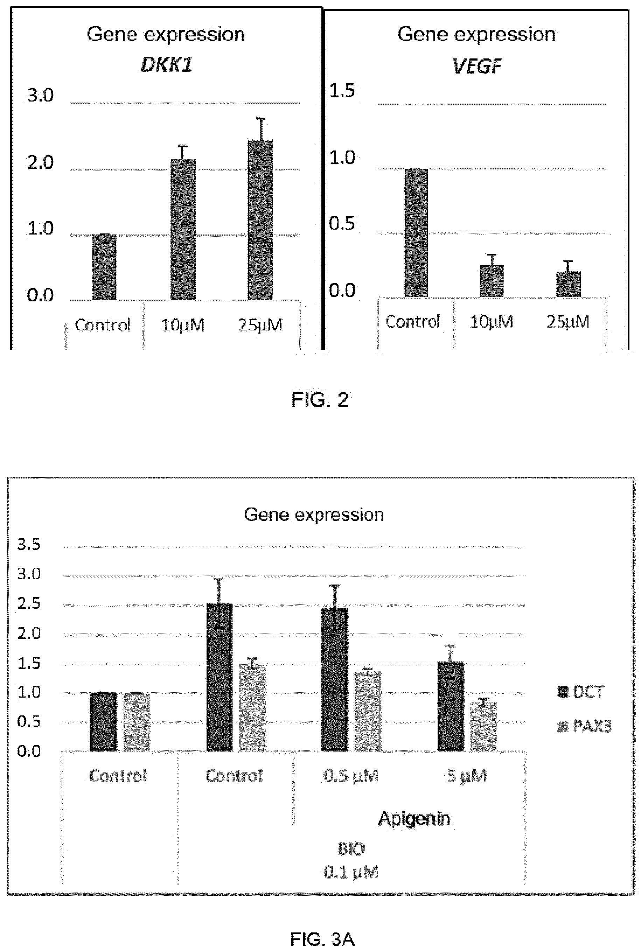

As can be observed in figure 2, the active ingredient apigenin significantly induces the DKK1 expression and reduces the VEGF expression. These results indicate that the active ingredient inhibits the Wnt pathway indirectly after increasing the DKK1 inhibitor, while also reducing angiogenesis and activation of melanogenesis due to the vascular component, being both processes related to hyperpigmentation pathologies (Lee AY. Recent progress in melasma pathogenesis. Pigment Cell Melanoma Res. 2015; 28(6): 648-60; Passeron T, Picardo M. Melasma, a photoaging disorder. Pigment Cell Melanoma Res. 2018; 31(4): 461-465).

### Example 3: Effect of apigenin and phloretin on the Wnt pathway in melanocytes

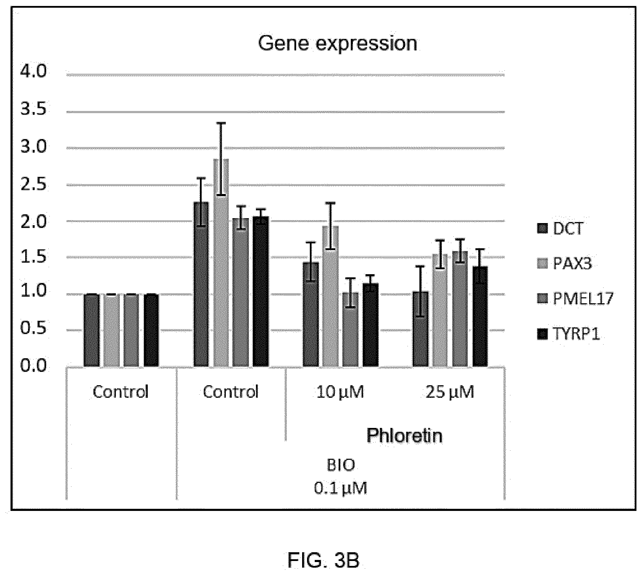

In order to assess the inhibition of the Wnt pathway in melanocytes, human epidermal melanocytes were cultured with 0.1 µM BIO ((3E)-6-bromo-3-[3-(hydroxylamine)indol-2-ilideno]-1H-indol-2-on, a Wnt pathway-activating molecule) + 0.5 and 5 µM apigenin or 10 and 25 µM phloretin for 24 hours. After the treatment, the RNA was extracted (Norgen, #17200) and a real-time qPCR was performed (Takara, #RR420A) on the TYRP1, DCT, PAX3 and PMEL17 genes. The results are shown in figure 3A and 3B.

As can be observed, in the Wnt pathway in melanocytes, the active ingredients apigenin and phloretin significantly reduce the expression of melanogenesis-regulating genes after stimulation with BIO (Wnt pathway-activating molecule).

### Example 4: Effect of apigenin and phloretin on the SCF pathway in melanocytes

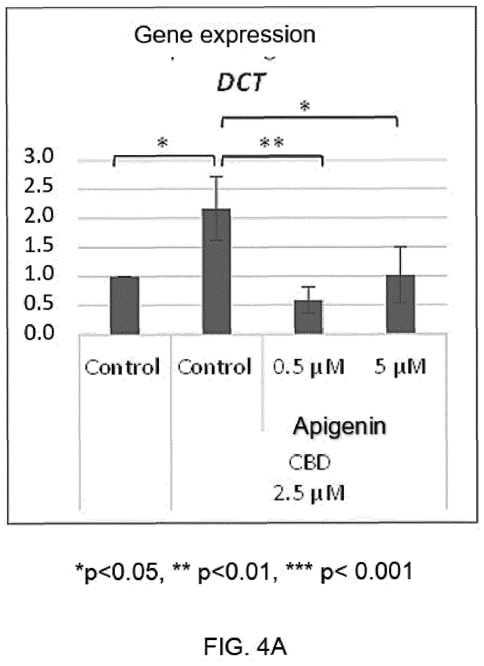

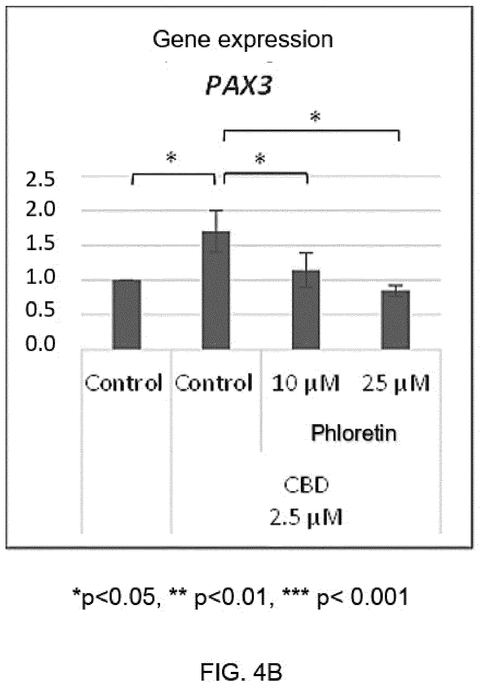

In order to assess the inhibition of the SCF pathway in melanocytes, human epidermal melanocytes were cultured with 2.5 µM cannabidiol (CBD) + 0.5 and 5 µM apigenin or 10 and 25 µM phloretin for 24 hours. After the treatment, the RNA was extracted (Norgen, #17200) and a real-time qPCR was performed (Takara, #RR420A) on the DCT and PAX3 genes. The results are shown in figures 4A and 4B.

As can be observed, in the SCF pathway in melanocytes, the active ingredients apigenin and phloretin significantly reduce the expression of the melanogenesis-regulating genes after stimulation with CBD.

### Example 5: Effect of apigenin and phloretin on miRNA levels in melanocytes

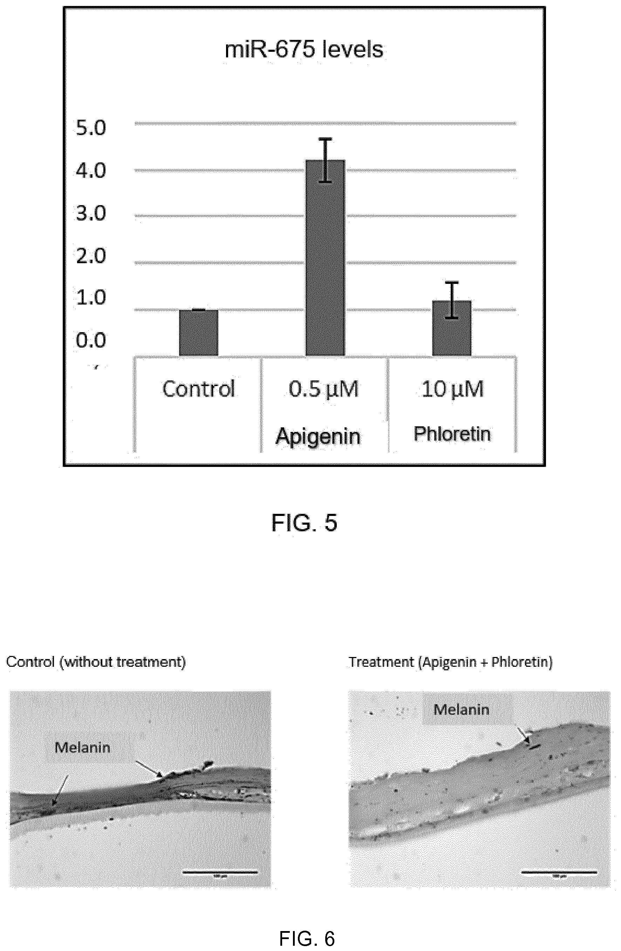

In order to assess the effect of miRNA levels in melanocytes, human epidermal melanocytes were cultured with 0.5 µM apigenin or 10 µM phloretin for 24 hours. After the treatment, the RNA was extracted (Norgen, #17200) and a real-time qPCR was performed (Takara, #638313) on miR-675. The results are shown in figure 5.

As can be observed, the active ingredient apigenin is capable of significantly increasing miR-675levels. This miRNA is reduced in melasma and inhibits pigmentation upon inhibiting the expression of the genes involved in melanogenesis (Kim N.H. and colleagues, Reduced MiR-675 in exosome in H19 RNA-related Melanogenesis via MITF as a direct target. J Invest Dermatol. 2014; 134(4): 1075-1082), due to which the increase thereof contributes to the depigmenting mechanism of apigenin.

### Example 6: Effect of the combination of apigenin and phloretin in an ex vivo model of the human epidermis

The depigmenting effectiveness of the active ingredients in *ex vivo* 3D models of reconstructed pigmented human epidermis (Fontana-Masson stain) phototype VI after applying the combination of apigenin and phloretin for a week was analysed. Different histological sections were analysed to obtain representative information on the effectiveness of the treatment. The results of the *ex vivo* experiments showed a reduction in the accumulations contrasted in black (corresponding to melanin) after treatment with the combination of apigenin and phloretin (Figure 6). As can be observed in the figure, the image on the left is a control without treatment and the image on the right shows the result after treatment with the combination of apigenin and phloretin. The areas contrasted in black correspond to melanin accumulations.

### Example 7: Cosmetic compositions

Formulation for the co-encapsulation of the combination of apigenin and phloretin in transethosomes:

| **Compound** | **Concentration (% by weight)** |
|---|---|
| Phosphatidyl choline | 0.1 - 8 |
| Ethanol | 5 - 50 |
| Activador Edge | 0.1 - 5 |
| Apigenin | 0.01 - 10 |
| Phloretin | 0.01 - 10 |
| Phenoxyethanol | 0.1 - 1.0 |
| Purified water | Up to 100 |

Formulation of the composition which includes the combination of apigenin and phloretin in cream form:

| | % by weight |
|---|---|
| Isopenthidiol, pentylene glycol, 1,2-hexanediol | 5 |
| Butylene glycol | 2 |
| Disodium EDTA | 0.1 |
| Phenoxyethanol-ethyl hexyl glycerin | 1 |
| Xanthan gum | 0.2 |
| Caprylic/capric acid triglyceride | 3 |
| Isononyl isonononanoate | 3 |
| Isodecyl neopentanoate | 3 |
| Undecane, Tridecane, Tocopherol, Sunflower oil | 5 |
| Cetearyl alcohol, glyceryl stearate, jojoba esters, sunflower seed wax, sodium stearylglutamate, polyglycerin-3 | 5 |
| Dimethicone | 2 |
| Cetyl alcohol | 2 |
| Propanediol, acetyl tetrapeptide-2 | 1 |
| PEG-8, Tocopherol, Ascorbyl palmitate, Ascorbic acid, Citric acid | 0.2 |
| Hydroxyethyl acrylate/sodium acryloyldimethyl sodium tartarate copolymer, squalene, polysorbate 60, sorbitan isostearate | 2 |
| Sodium hydroxide | 0,2 |
| Apigenin | 0.1-0.5 |
| Phloretin | 0.05-2 |
| Perfume | 0.3 |
| Water | Up to 100 |

Formulation of the composition which includes the combination of apigenin and phloretin in serum form:

| | **% by weight** |
|---|---|
| Butylene glycol | 10 |
| Propylene glycol | 10 |
| Isopenthidiol, pentylene glycol, 1,2-hexanediol | 5 |
| Phenoxyethanol-ethyl hexyl glycerin | 1 |
| Sodium acrylate crosspolymer-2 | 5 |
| Disodium EDTA | 0.1 |
| Trideceth-9, hydrogenated castor oil PEG-40, polysorbate 20 | 1.5 |
| Sodium hydroxide | 0.2 |
| Apigenin | 0.1-0.5 |
| Phloretin | 0.05-2 |
| Propanediol, Acetyl tetrapeptide-2 | 1 |
| Perfume | 0.2 |
| Water | Up to 100 |

## Claims

1. A cosmetic composition having a skin depigmenting effect which includes, as sole active ingredient, a combination of apigenin and phloretin, wherein the components of said combination act synergistically, together with the appropriate carriers, solvents and/or excipients for the topical administration thereof.

2. The cosmetic composition having a skin depigmenting effect, according to claim 1, **characterised in that** the components apigenin and phloretin are present in the combination in an apigenin:phloretin ratio of 1:0.05 to 1:10 (by weight).

3. The cosmetic composition having a skin depigmenting effect, according to claim 1, **characterised in that** the combination of apigenin and phloretin is present in the cosmetic composition in a proportion by weight, with respect to the total weight of the composition, between 0.02 and 20%.

4. A combination of apigenin and phloretin for use in the manufacture of a cosmetic composition for treating conditions related to the overproduction or uneven distribution of melanin, wherein said use comprises topically administering an effective quantity of said combination to a patient who needs it.

5. The combination of apigenin and phloretin for the use thereof, according to claim 4, **characterised in that** the apigenin:phloretin ratio is 1:0.05 to 1:10 (by weight).
